# EUROPEAN PATENT APPLICATION

(11) **EP 3 251 632 A1**
(43) Date of publication of application: **06.12.2017**
(21) Application number: 16743561.9
(22) Date of filing: 29.01.2016
(51) Int. Cl.: A61F 2/20, A61M 16/04

(54) **SPEAKING VALVE**

(30) Priority: 30.01.2015 JP 2015016803
(71) Applicant: Senko Medical Instrument Mfg. Co., Ltd., Tokyo 113-0033 (JP)
(72) Inventor: KANAZAWA, Hideaki, Hamamatsu Shizuoka 433-8511 (JP); CHIBA, Mitsuru, Tokyo 113-0033 (JP); OKADA, Tomoaki, Tokyo 113-0033 (JP); ABE, Chinami, Tokyo 113-0033 (JP)
(74) Representative: Beck Greener
(86) International application number: PCT/JP2016/052738
(87) International publication number: WO 2016/121958

(57) **Abstract**

A speaking valve (1A) which is applied to a respiration opening (101) of a tracheostomy tube (100) comprises: a main body portion (2) which has a passage portion (3) communicating with the respiration opening (101); a balloon (3) which makes a motion between an opened state for opening the passage portion (3) of the main body portion (3) and a closed state for closing the passage portion (3); a syringe (5) which is provided away from the main body portion (2) and accepts a pushing operation to a plunger 5d by a patient; and a coupling tube (6) which couples the syringe (5) and the balloon (4) with each other and makes the balloon (4) in a shrink state transform to a bloat state by the operation to the syringe (5), so that the motion from the opened state to the closed state is made.

## Description

### Technical Field

The present invention relates to a speaking valve which enables a patient to produce speech.

### Background Art

There is known a speaking valve which is applied to a respiration opening of a tracheostomy tube for enabling a patient into whom a tracheostomy tube is inserted to produce speech. As the speaking valve, there is known a type of a one-way valve which allows airflow of the respiration opening to head for the inside of the body of the patient from the outside thereof while inhibiting airflow of the respiration opening from heading for the outside of the body of the patient from the inside thereof. In addition, there is known another type of speaking valve which is configured so that the patient is required to close an opening which communicates with the respiration opening of the tracheostomy tube with his/her fingers when he/she produces speech (for example, the patent literature 1) .

### Citation List

### Patent Literature

PTL1: JP-B-4166946

### Summary of Invention

### Technical Problem

Since it is inevasible that the speaking valve configured as the one-way valve becomes a hindrance when a patient is breathing, the patient sometimes has to suffer from difficulty breathing. Within patients into whom a tracheostomy tube is inserted, there are some patients, for example, whose arms swing despite their intentions. In this way, there are not a little patients who cannot move their arms as their intentions. In a case that the speaking valve requiring fingers to close the opening is used to such a patient, since it is difficult for the patient to move his/her hand up to his/her throat and close the opening with his/her fingers, there is such a trouble that nursing care is required for speech production.

Then, the present invention aims to provide a speaking valve whose opening is opened and closed by a simple operation by a patient.

### Solution to problem

One aspect of the present invention provides a speaking valve which is applied to a respiration opening of a tracheostomy tube, comprising: a main body portion which has a passage portion communicating with the respiration opening; a valve portion which is allowed to make a motion between an opened state where the valve portion is in a state for opening the passage portion of the main body portion and a closed state where the valve portion is in a state for closing the passage portion; an operation portion which is provided away from the main body portion and accepts a predetermined operation performed by a patient; a motion converting portion which couples the operation portion and the valve portion with each other, and converts the predetermined operation performed to the operation portion to a motion made by the valve portion from the opened state to the closed state.

According to this speaking valve, by the operation performed by the patient to the operation portion provided away from the main body portion, the valve portion makes a motion from the opened state for opening the passage portion to the closed state for closing the passage portion. Thereby, a patient into whom the tracheostomy tube is inserted is not required to move his/her hand up to his/her throat to close the opening with his/her fingers, and it is possible to produce speech by the operation by the patient performed away from the main body portion. Accordingly, even if the patient is difficult to close with his/her fingers the opening on his/her throat, he/she is enabled to produce speech. As the predetermined operation which is accepted by the operation portion, based on assumption of a state of a patient as an object, a simple operation could be employed as appropriate, such as gripping the operation portion by hand and pushing the operation portion with fingers.

In one embodiment of the speaking valve of the present invention, a balloon may be provided as the valve portion, the balloon being disposed in the passage portion of the main body portion, and transforming from a shrink state to a bloat state by air supplied to a supply opening so as to make the motion from the opening state to the closed state, a manual pump may be provided as the operation portion, the manual pump discharging air from a discharge opening by the predetermined operation, and a coupling tube may be provided as the motion converting portion, the coupling tube coupling the discharge opening of the manual pump and the supply opening of the balloon with each other in an airtight state.

According to this embodiment, the air discharged by the manual pump is supplied to the balloon via the coupling tube, thereby the balloon in the shrink state transforms to the bloat state to close the passage portion. Since the coupling tube couples the discharge opening of the manual pump and the supply opening of the balloon with each other in an airtight state, when the predetermined operation to the manual pump is released, the air supplied to the balloon returns to the manual pump, thereby the balloon in the bloat state transforms to the shrink state to open the passage portion. That is, when the patient releases the predetermined operation to the manual pump, thereby the passage portion is opened. Due to this, the risk that the passage portion is held in the closed state is made small.

With respect to the above embodiment, each portion may be defined as follows. A dividing portion may be provided to the main body portion so as to zone the passage portion into a main region and an air region smaller than the main region, and the balloon may be disposed in the main region generated by the dividing portion. Alternatively, a contact inhibition portion may be formed in the passage portion of the main body portion, the contact inhibition portion inhibiting contact of the balloon and the passage portion in such a way that, when the balloon is in the bloat state, the passage portion is not completely closed so as to make an airway. Alternatively, a discharge amount of air which is discharged from the manual pump by the predetermined operation may be controlled so that, when the balloon is in the bloat state, the passage portion of the main body portion is not completely closed so as to make an airway. According to the above definitions, even if the balloon transforms from the shrink state to the bloat state, the passage potion is not closed completely, and thereby the breathability is ensured by the air region or the airway which is formed in the passage portion. Due to this, its safety is improved. The degree of the breathability may be set as appropriate within a range that speech production is not hindered.

In one embodiment of the above speaking valve of the present invention, a valve body may be provided as the valve portion in the passage portion of the main body portion, the valve body being provided so as to make a motion between the opened state and the closed state, the operation portion may have a displacement portion which displaces by the predetermined operation, and a wired mechanism may be provided as the motion converting portion, the wired mechanism transmitting a displacement of the displacement portion of the operation portion so as to convert the displacement to the motion made by the valve body from the opened state to the closed state.

According to this embodiment, the displacement portion is displaced by the predetermined operation accepted to the operation portion. The displacement of the displacement portion is converted by the wired mechanism to the motion made by the valve body from the opened state to the closed state. Thereby, the passage portion is closed. In this way, by the operation to the operation portion positioned away from the main body portion, the patient is enabled to produce speech.

As one embodiment of the above speaking valve of the present invention, the passage portion or the valve portion may be configured so that, when the valve portion is in the closed state, the passage portion is not completely closed in order to ensure breathability. According to this embodiment, even if the valve portion makes a motion from the opened state to the closed state, the passage potion is not closed completely, and thereby the breathability is ensured. Due to this, the safety is improved. The degree of the breathability may be set as appropriate within a range that speech production is not hindered.

In the above descriptions, referential symbols of attached drawings are noted in brackets for making the present invention comprehensible. However, it should be understood that the present invention is not limited to the embodiment shown in drawings.

### Brief Description of Drawings

Fig.1 is a diagram showing a tracheostomy tube to which a speaking valve according to a first embodiment of the present invention is applied;
Fig.2 is a perspective view showing a state of the tracheostomy tube viewed in a direction of an arrow II shown in fig.1;
Fig.3 is a diagram showing a state of the speaking valve viewed in a direction of an arrow III shown in fig.1;
Fig.4 is an exploded perspective view of the speaking valve;
Fig.5A is a diagram showing a first other embodiment of the valve portion;
Fig.5B is a diagram showing a second other embodiment of the valve portion;
Fig.6 is a diagram showing a main portion of the speaking valve according to a second embodiment of the present invention;
Fig.7 is a diagram showing a main portion of the speaking valve according to a third embodiment of the present invention;
Fig.8 is a diagram showing the other embodiment of the operation portion; and
Fig.9. is a diagram showing an embodiment where a coiled spring is attached to a syringe.

### Description of Embodiments

### (First Embodiment)

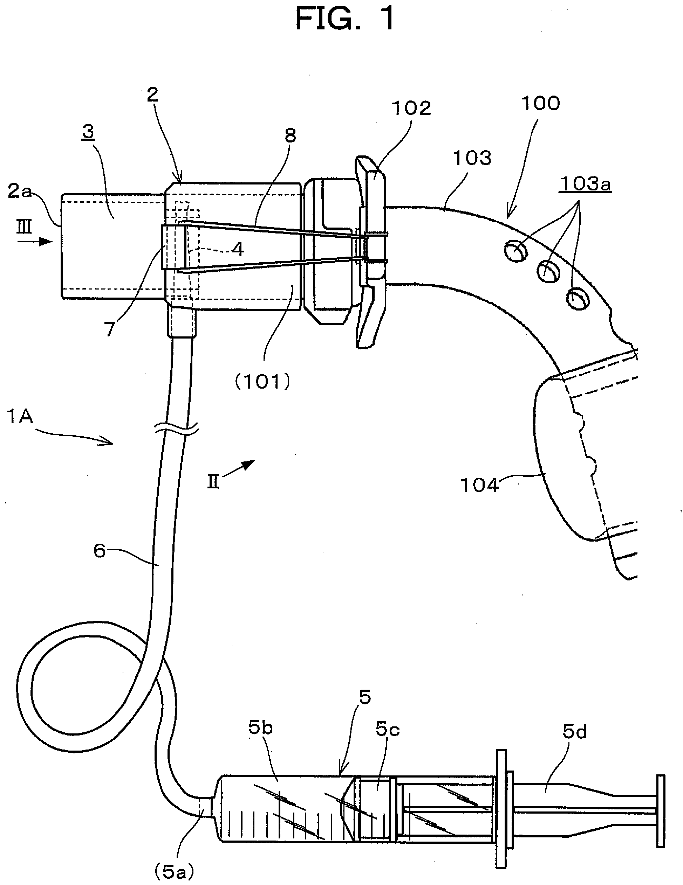

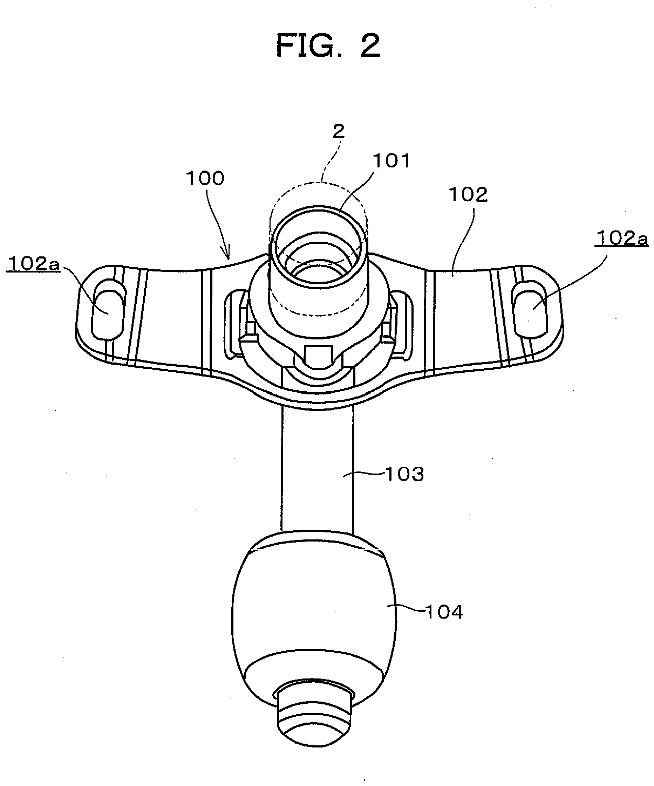

As shown in figs.1 and 2, a speaking valve 1A is applied to a respiration opening 101 of a tracheostomy tube 100. The respiration opening 101 has a diameter allowing a pipe of a general artificial respirator (not illustrated) to connect to the respiration opening. A main body portion 2 of the speaking valve 1A is formed as a connector allowed to connect to the respiration opening 101, and an opening end 2a of the main body portion 2 has a diameter equivalent to the diameter of the respiration opening 101. Accordingly, the pipe of the artificial respirator is allowed to connect to the opening end 2a of the main body portion 2 to which the respiration opening 101 is connected.

The tracheostomy tube 100 has a frame 102 for fixing, with tape or the like not illustrated, the tracheostomy tube 100 to a throat portion of a patient to whom the tracheostomy tube 100 is inserted and a pipe portion 103 which is inserted into the trachea of the patient. As shown in fig.2, the frame 102 extends in a right-left direction from the pipe portion 103, and at an each end of the frame 102, an opening 102a where the not-illustrated tape could be twisted is formed. In the pipe portion 103, a plurality of side eyelets 103a are formed for introducing air to a vocal-cords side of the patient, for a case that the respiration opening 101 is closed by the speaking valve 1A or the like. Each of the plurality of side eyelets 103a is formed so as to pass through the inside and outside of the pipe portion 103. At a lower portion of the side eyelets 103a, a sealing portion 104 is provided for bringing the pipe portion 103 into close contact with an inside of the trachea of the patient in order to prevent air leak at an outer-circumference side of the pipe portion 103.

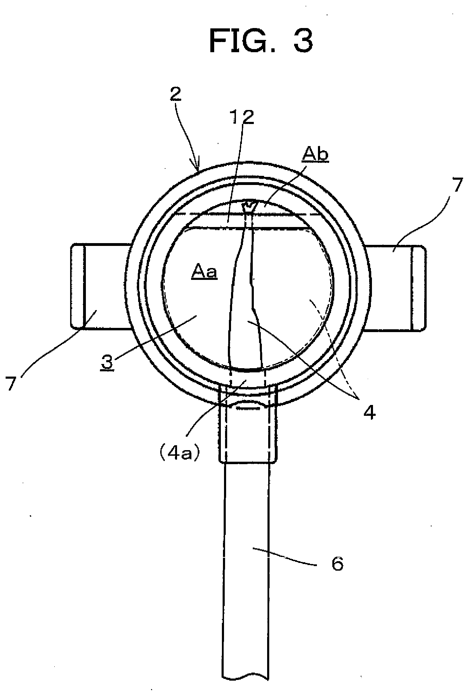

As shown in fig.1, the main body portion 2 of the speaking valve 1A has a passage portion 3 which communicates with the respiration opening 101 while the main body portion 2 is connected to the respiration opening 101 of the tracheostomy tube 100. As shown in figs.1 and 3, the speaking valve 1A is provided with: a balloon 4, a syringe 5, and a coupling tube 6. The balloon 4 is provided as a valve portion which is allowed to make a motion between an opened state where the passage portion 3 is opened and a closed state where the passage portion 3 is closed. The syringe 5 is provided apart from the main body portion 2 as an operation portion which is operated by the patient. The coupling tube 6 is provided as a motion converting portion which couples the balloon 4 and the syringe 5 with each other and converts the operation to the syringe 5 to the motion of the balloon 4. For preventing misoperation of a nurse, it is preferable that the coupling tube 6 is colored. Further, the coupling tube 6 has a length sufficient for preventing the main body portion 2 from being pulled even if a patient moves his/her arm because of convulsion or the like. In order not to uncouple easily the main body portion 2 from the tracheostomy tube 100, at each side of the main body portion 2, a protrusion 7 is provided for preventing the uncoupling. In addition, as shown in fig.1, provided is an elastic band 8 one end of which is passed through the opening 102a of the frame 102 and tied to the frame 102. The other end of the elastic band 8 is allowed to be hooked to the protrusion 7 of the main body portion 2 in a state that the main body portion 2 is attached to the tracheostomy tube 100. Thereby, it is realized to prevent the main body 2 from uncoupling from the tracheostomy tube 100.

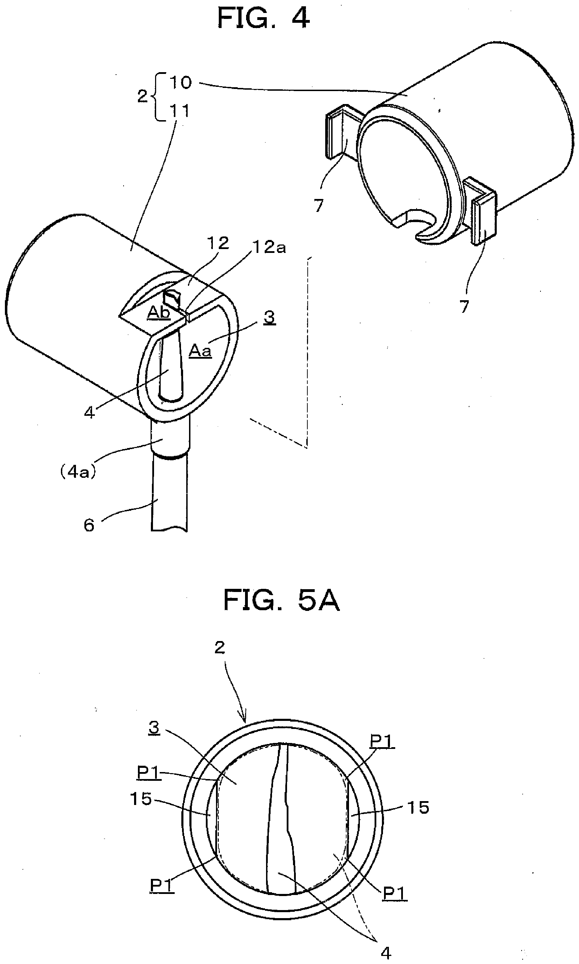

As shown in fig.3, the balloon 4 is formed in a cylindrical shape. One end of the balloon 4 is fixed to the main body portion 2 in a closed state and the other end is formed as a supply opening 4a where air is supplied (see also fig.4). By supply of air to the supply opening 4a of the balloon 4, the balloon 4 transforms from a shrink state shown by a solid line to a bloat state shown by a chain double-dashed line. Thereby, the balloon 4 makes a motion between the opened state where the passage portion 3 is opened to the closed state where the passage portion 3 is closed. In light of security, the passage portion 3 is not closed completely by the balloon 4, so that breathability is secured to a degree that speech production is not hindered. Details thereof will be described later. In order to simplify a check of taint on the balloon 4, it is preferable that the balloon 4 is translucent or colored pale color such as white. Further, it is preferable that the balloon 4 is made of latex-free materials.

The syringe 5 is a kind of a manual pump which is distributed as a general production. The syringe 5 comprises a main body 5b where a discharge opening 5a is formed and a plunger 5d which is inserted into the main body 5b and provided with a gasket 5c at the tip thereof. When the plunger 5d of the syringe 5 is pushed into the main body 5b, thereby air between the main body 5b and the gasket 5c is discharged from the discharge opening 5a.

The coupling tube 6 couples the supply opening 4a of the balloon 4 and the discharge opening 5a of the syringe 5 with each other in an airtight state. Due to this, when the plunger 5d of the syringe 5 is pushed as a predetermined operation of a patient, thereby air discharged from the discharge opening 5a of the syringe 5 is supplied into the balloon 4 through the coupling tube 6. Because of this, the balloon 4 transforms from the shrink state to the bloat state to close the passage portion 3, thereby the patient is allowed to produce speech. And then, when the pushing operation to the plunger 5d by the patient is released, the air supplied to the balloon 4 returns to syringe 5. Because of this, the balloon 4 transforms from the bloat state to the shrink state to open the passage portion 3. In this way, in the case of the present embodiment, when the patient leaves his/her hand from the syringe 5, the pushing operation to the plunger 5d is released. Because of this, the risk that the passage portion 3 is held in the closed state is made small.

As shown in details in fig.4, the main body portion 2 is formed in such a way that a female member 10 where the respiration opening 101 of tracheostomy tube 100 is connected and a male member 11 where the balloon 4 is provided are coupled with each other. By making the outer circumference of the male member 11 engage with the inner circumference of the female member 10, the passage portion 3 is formed.

As comprehensibly shown by the male member 11 in fig.4, the passage portion 3 is zoned by a dividing portion 12 provided in the mail member 11, into a main region Aa and an air region Ab smaller than the main region Aa (please see also fig.3). In the dividing portion 12, formed is a slit portion 12a for fixing one end of the balloon 4. When the one end of the balloon 4 is engaged with the slit 12a, thereby the one end of the balloon 4 is fixed in a closed state. Thereby, the balloon 4 is disposed in the main region Aa with the one end closed. Due to this, even if the balloon 4 has transformed to the bloat state, without closing completing the passage portion 3, breathability is ensured by the air region Ab generated by the dividing portion 12. Accordingly, its safety is improved. The size of the air region Ab may be set as appropriate within a range that speech production is hindered.

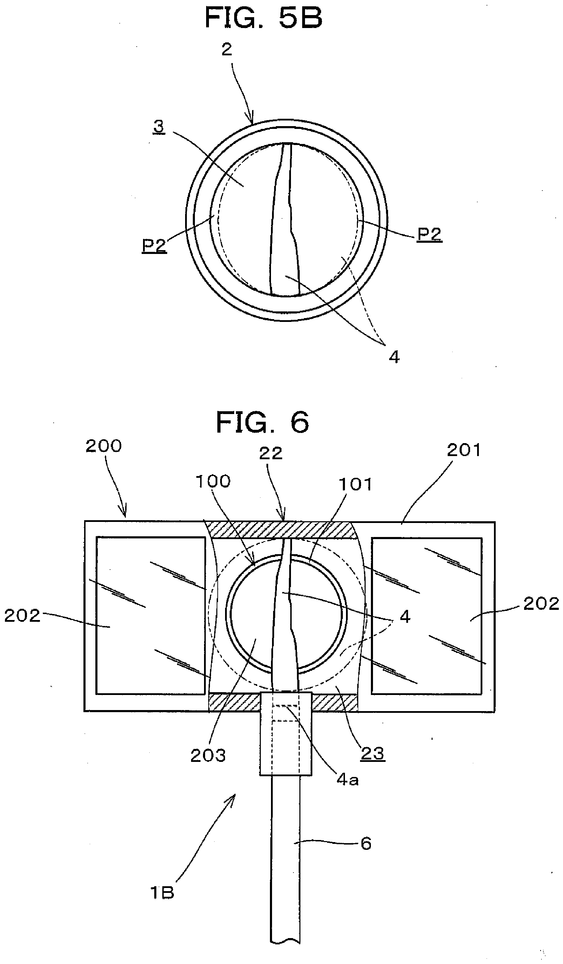

As mentioned above, in the present embodiment, the breathability is ensured by the air region Ab generated by the dividing portion 12. However, even if the embodiment of the speaking valve 1A is changed to the other embodiments shown in figs.5A and 5B, it is also possible to ensure the breathability in the bloat state of the balloon 4. In the embodiment shown in fig.5A, the passage portion 3 is provided with a convex portion 15 as a contact inhibition portion which inhibits a close contact of the balloon 4 and the passage portion 3 when the balloon 4 has transformed to the bloat state. The convex portion 15 is formed so as to protrude from the inner wall of the passage portion 3 toward the center of the passage portion 3. Thereby, even if the balloon 4 has transformed to the bloat state, as shown by a chain double-dashed line, an airway P1 is ensured between the convex portion 15 and the passage portion 3.

On the other hand, in the embodiment shown in fig.5B, a bloat degree of the balloon 4 is controlled by restricting the amount of air discharged by the syringe 5. In the case shown in fig.5B, as shown by a chain double-dashed line, when the balloon 4 has transformed to the bloat state, a supply amount of air is restricted so that an airway P2 is formed without completely closing the passage portion 3. The restriction of the air amount discharged by the syringe 5 is realized by a method that a movement amount of the plunger 5d is restricted, a method that the air amount held by the syringe 5 is restricted in advance, or the like.

According to the speaking valve 1A mentioned above, when a patient pushes the plunger 5d of the syringe 5 provided away from the main body portion 2, thereby the balloon 4 in the shrink state transforms to the bloat state so as to close the passage portion 3. Thereby, a patient into whom the tracheostomy tube 100 is inserted is not required to move his/her hand up to his/her throat to close the opening with his/her fingers, and it is possible to produce speech by the operation by the patient performed away from the main body portion 2. Accordingly, even the patient, who is difficult to close with his/her fingers the opening on his/her throat, is enabled to produce speech.

### (Second Embodiment)

Next, a second embodiment will be described with fig.6. Hereinafter, each member shard with the first embodiment is assigned to the same referential sign in fig.6, and the description thereof will be omitted. In the second embodiment, a speaking valve 1B comprises a main body portion 22, and the main body portion 22 is built in a heat moisture exchanger ("HME") 200 which is connectable to the tracheostomy tube 100. As well known, the HME 200 has a cylindrical chamber 201 which has opening portions at both sides thereof respectively, and a filter 202 is attached to each of the opening portions provided each side of the chamber 201. At the central portion of the chamber 201, a connection opening 203 is provided for connecting to the respiration opening 101 of the tracheostomy tube 100. While the HME 200 is connected to the respiration opening 101, the inside of the chamber 201 is communicated with the respiration opening 101. Accordingly, a part of the chamber 201 corresponds to the main body portion 22 of the speaking valve 1B, and the inside of the chamber 201 corresponds to the passage portion 23 which is provided to the main body portion 22.

The passage portion 23 is provided with the balloon 4. As with the first embodiment, the coupling tube 6 is coupled with the supply opening 4a of the balloon 4, and the other end of the coupling tube 6 is connected to the discharge opening 5a of the syringe 5, the illustration of which is omitted in fig.6 (please see fig.1). Accordingly, when the patient performs the pushing operation to the plunger 5d of the syringe 5, thereby the balloon 4 transforms from the shrink state shown by a solid line to the bloat state shown by a chain double-dashed line to close the passage portion 3. Thereby, as with the first embodiment, the patient into whom the tracheostomy tube 100 is inserted is enabled to produce speech.

### (Third Embodiment)

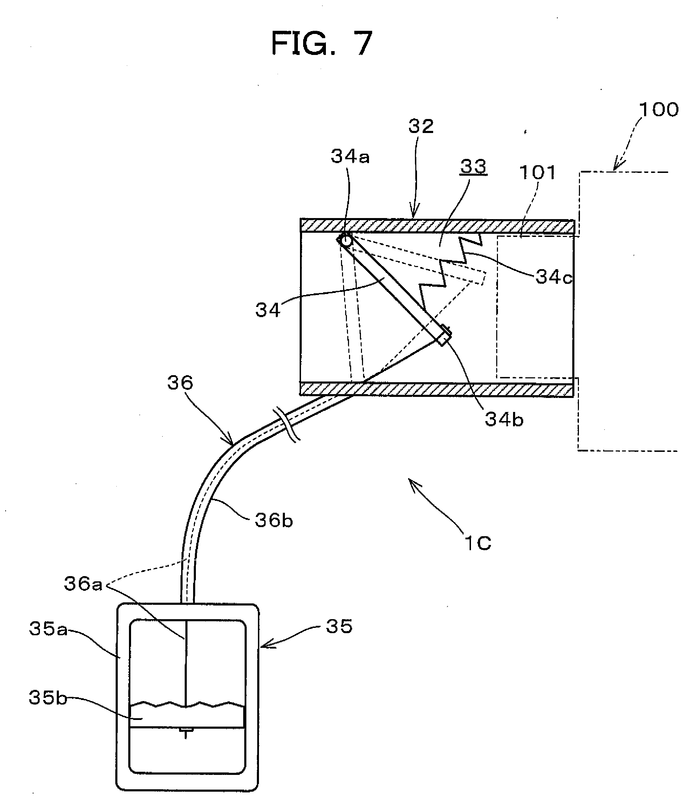

Next, a third embodiment will be described with fig.7. Hereinafter, each member shard with the first embodiment is assigned to the same referential sign in fig.7, and the description thereof will be omitted. In the third embodiment, a speaking valve 1C comprises: a main body portion 32 which is connected with the respiration opening 101 of the tracheostomy tube 100; a valve body 34 as the valve portion for open and close of a passage portion 33 of the main body portion 32, the passage portion 33 communicating with the respiration opening 101; a manually operated unit 35 as the operation portion provided away from the main body portion 32; and a wired mechanism 36 as the motion converting portion which couples the valve body 34 and the manually operated unit 35 with each other.

The valve body 34 is attached to the main body portion 32 so that one end 34a of the vale body 34 is freely rotatable, and the valve body 34 is biased so as to return to the opened state shown by a broken line by a returning spring 34c as a biasing device. When the valve body 34 operates from the opened state shown by the broken line to the closed state shown by the chain double-dot line, thereby the passage portion 33 is closed. The manually operated unit 35 has an outer frame 35a; and a grip 35b as a displacement portion provided slidably inside the outer frame 35a. The wired mechanism 36 comprises: a wire 36a which couples the other end 34b of the valve body 34 and the grip 35b of the manually operated unit 35 with each other; and an outer cable 36b covering around the wire 36a where one end is fixed to the main body portion 32 and the other end is fixed to the outer frame 35a of the manually operated unit 35.

By the speaking valve 1C having the above configuration, when a patient grips the grip 35a of the manually operated unit 35 as the predetermined operation by the patient, thereby the grip 35b is displaced, and the displacement is transmitted to the valve body 34 through the wire 36a. Because of this, the displacement is converted to the motion of the valve body 34 for making the valve body 34 transform from the opened state shown by the broken line to the closed state shown by the chain double-dot line. And then, when the patient releases the grip 35a, the valve body 34 returns to the opened state from the closed state by elastic force of the returning spring 34c.

According to the speaking valve 1C of the third embodiment, as with the first embodiment, by the operation by the patient such that the patient grips the grip 35b of the manually operated unit 35 provided away from the main body portion 32, the valve body 34 in the opened state make a motion so as to change its state to the closed state to close the passage portion 33. Thereby, a patient into whom the tracheostomy tube 100 is inserted is not required to move his/her hand up to his/her throat to close the opening with his/her fingers, and the patient is enabled to produce speech by the operation performed by the patient away from the main body portion 32. Accordingly, even the patient, who is difficult to close with his/her fingers the opening on his/her throat, is enabled to produce speech.

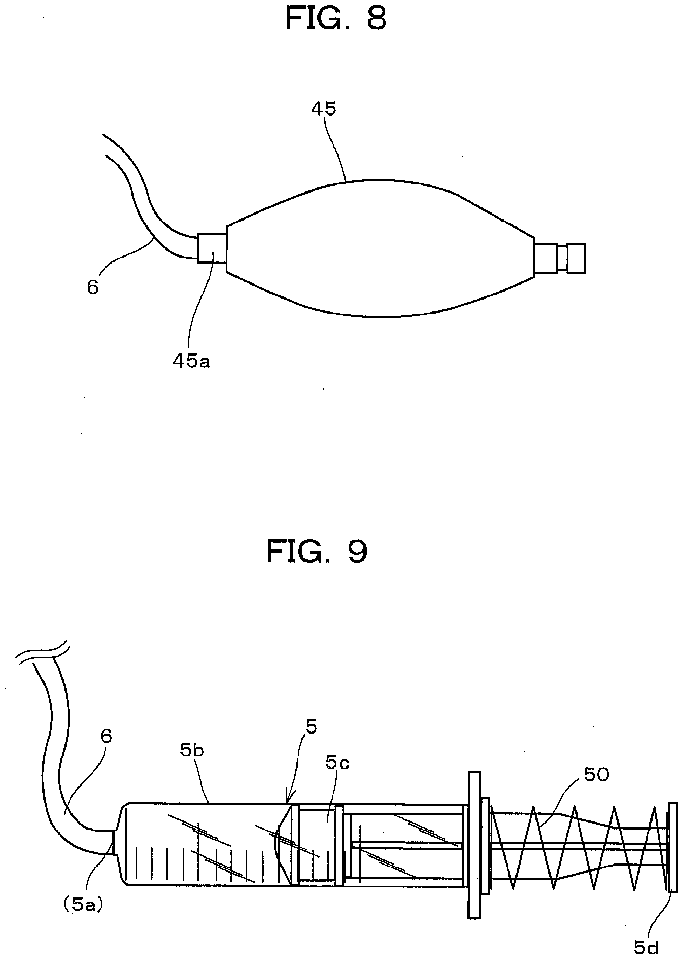

The present invention is not limited to the above embodiments, and may be executed in various embodiments. In the first and second embodiments, as one example of the manual pump which functions as the operation portion, the syringe 5 (fig.1) is employed. However, the embodiments may be executed with a well-known rubber-bulb pump 45 shown in fig.8 instead of the syringe 5. In a case that the syringe 5 is replaced with the rubber-bulb pump 45, the coupling tube 6 is connected to the discharge opening 45a of the rubber-bulb pump 45.

Further, as shown in fig.9, the present invention may be executed by employing as an embodiment that the plunger 5d is prevented from being held in a pushed state, an embodiment such that an elastic member, such as coiled spring 50, for return to the initial position intervenes between the main body 5b and plunger 5d of the syringe 5.

In the first embodiment, the opening end 2a of the main body portion 2 has a diameter which is suitable for connecting to the pipe of the artificial respirator. Due to this, the first embodiment provides a merit that the artificial respirator is allowed to be connected to the main body portion 2 in a state the speaking valve 1A is connected to the tracheostomy tube 100. However, the diameter of the main body portion 2 may be set in an arbitrary manner. For example, if a diameter larger than the diameter of the first embodiment is applied to the opening end 2a, it is possible to reduce resistance to breathing of the patient.

As the valve portion, the balloon 4 is employed in the first and second embodiments, and in the third embodiment, the valve body 34 is employed. These embodiments are just exemplifications of the valve portion. As long as the valve portion and the operation portion are non-electrically coupled with each other, it is possible to execute the present invention in various embodiments.

In the first and second embodiments, during the closed state the passage portion is not closed completely so that the breathability is ensured. Also, even in the third embodiment, in order to ensure the breathability similarly, for example, at least one hole may be formed in the valve body 34, or at least one groove may be provided to the passage portion 33. Thereby, the breathability can be ensured. However, it is not indispensable to ensure the breathability during the closed state. The present invention may be executed in an embodiment such that the passage portion is completely closed during the closed state.

In each of the above embodiments, an operation performed by hand is assumed as the predetermined operation to the operation portion by the patient. However, the present invention can also be executed with an operation portion configured so as to accept operations performed by any parts of the patient, for example, arms, legs, or feet, besides hands.

## Claims

1. A speaking valve which is applied to a respiration opening of a tracheostomy tube, comprising:
a main body portion which has a passage portion communicating with the respiration opening;
a valve portion which is allowed to make a motion between an opened state where the valve portion is in a state for opening the passage portion of the main body portion and a closed state where the valve portion is in a state for closing the passage portion;
an operation portion which is provided away from the main body portion and accepts a predetermined operation performed by a patient;
a motion converting portion which couples the operation portion and the valve portion with each other, and converts the predetermined operation performed to the operation portion to a motion made by the valve portion from the opened state to the closed state.

2. The speaking valve according to Claim 1, wherein
a balloon is provided as the valve portion, the balloon being disposed in the passage portion of the main body portion, and transforming from a shrink state to a bloat state by air supplied to a supply opening so as to make the motion from the opening state to the closed state,
a manual pump is provided as the operation portion, the manual pump discharging air from a discharge opening by the predetermined operation, and
a coupling tube is provided as the motion converting portion, the coupling tube coupling the discharge opening of the manual pump and the supply opening of the balloon with each other in an airtight state.

3. The speaking valve according to Claim 2, wherein
a dividing portion is provided to the main body portion so as to zone the passage portion into a main region and an air region smaller than the main region, and
the balloon is disposed in the main region generated by the dividing portion.

4. The speaking valve according to Claim 2, wherein
a contact inhibition portion is formed in the passage portion of the main body portion, the contact inhibition portion inhibiting contact of the balloon and the passage portion in such a way that, when the balloon is in the bloat state, the passage portion is not completely closed so as to make an airway.

5. The speaking valve according to Claim 2, wherein
a discharge amount of air which is discharged from the manual pump by the predetermined operation is controlled so that, when the balloon is in the bloat state, the passage portion of the main body portion is not completely closed so as to make an airway.

6. The speaking valve according to Claim 1, wherein
a valve body is provided as the valve portion in the passage portion of the main body portion, the valve body being provided so as to make a motion between the opened state and the closed state,
the operation portion has a displacement portion which displaces by the predetermined operation, and
a wired mechanism is provided as the motion converting portion, the wired mechanism transmitting a displacement of the displacement portion of the operation portion so as to convert the displacement to the motion made by the valve body from the opened state to the closed state.

7. The speaking valve according to Claim 1, wherein
the passage portion or the valve portion is configured so that, when the valve portion is in the closed state, the passage portion is not completely closed in order to ensure breathability.
